# FASCICULE DE BREVET EUROPEEN

(11) **EP 0 947 170 B1**
(45) Date de publication et mention de la délivrance du brevet: **03.12.2003**
(21) Numéro de dépôt: 99810254.5
(22) Date de dépôt: 22.03.1999
(51) Int. Cl.: A61B 17/16

(54) **Fraise chirurgicale**
Chirurgische Reibahle
Surgical reamer

(30) Priorité: 02.04.1998 CH 79398
(43) Date de publication de la demande: 06.10.1999
(73) Titulaire: PRECIMED S.A., 2534 Orvin (CH)
(72) Inventeur: Lechot, André, 2534 Orvin (CH)
(74) Mandataire: Meylan, Robert Maurice

(56) Documents cités:
- DE-U- 7 113 620
- US-A- 3 633 583
- US-A- 5 290 315

## Description

La présente invention a pour objet une fraise chirurgicale, destinée en particulier au façonnage de la cavité cotyloïdienne lors d'un remplacement par prothèse totale de l'articulation de la hanche, en forme de corps creux découpé dans un corps de révolution et muni de moyens de fixation à un porte-fraise pour son entraînement en rotation.

Du brevet FR 2 281 095, on connaît une fraise chirurgicale en forme de calotte hémisphérique munie d'une barre diamétrale pour sa fixation sur un porte-fraise pour son entraînement en rotation autour d'un axe coïncidant avec l'axe de symétrie de la calotte hémisphérique. La surface de la calotte est pourvue de nombreuses dents de coupe formées par perçage et repoussage vers l'extérieur du métal de la calotte. Cette conception, rappelant une râpe à légumes, a été reprise dans des exécutions postérieures, telles que les exécutions décrites dans les demandes de brevets EP 0 704 191 et EP 0 782 840.

De telles fraises assurent un fraisage efficace, mais ne permettent pas d'obtenir une cavité de grande précision. Or, la tendance actuelle dans la pose de prothèses est de ne plus utiliser ni ciment, ni vis. Il serait par conséquent souhaité de pouvoir façonner la cavité cotyloïdienne de façon beaucoup plus précise et plus lisse qu'on peut le faire au moyen des fraises connues.

La présente invention a pour but de réaliser une fraise chirurgicale permettant de façonner de façon précise une cavité hémisphérique ou autre forme.

La fraise selon l'invention est caractérisée en ce que le corps creux est supporté par une barre dont l'axe coïncide au moins approximativement avec l'axe de rotation de la fraise, que le corps creux s'étend essentiellement d'un côté d'un plan contenant l'axe de ladite barre et qu'une partie au moins de la longueur de son bord forme une arête de coupe.

Selon un mode particulier d'exécution, le corps creux est en forme de calotte hémisphérique. Une telle fraise est particulièrement destinée au façonnage de la cavité cotyloïdienne de la hanche.

Une surface sphérique pourrait cependant également être fraisée par une calotte en forme de secteur de sphère.

La calotte hémisphérique sera généralement orientée de manière à tourner autour d'un diamètre, mais dans certains cas la calotte pourra être légèrement inclinée relativement à l'axe de rotation, de telle sorte que cet axe de rotation n'est plus dans le plan méridien limitant la calotte hémisphérique.

Le bord du corps creux pourrait présenter une arête tranchante sur une longueur supérieure à la moitié de sa longueur, en particulier, pour une calotte hémisphérique, sur une longueur supérieure à la moitié de la circonférence de son bord.

La surface extérieure de la fraise est, de préférence, parfaitement lisse et rectifiée, ce qui permet de façonner une cavité osseuse lisse et précise. Pour certaines applications, la surface de la fraise pourrait cependant présenter au moins une dent de coupe et d'appui analogue aux dents des fraises de l'art antérieur et/ou au moins un dôme d'appui de manière à dégager un espace en arrière de l'arête de coupe, afin d'éviter un bourrage.

Selon un mode d'exécution à calotte hémisphérique, le bord de la calotte présente au moins un décrochement situé dans le plan équatorial de la sphère reconstituée par la rotation de la calotte. Ce décrochement permet de visualiser la pénétration de la fraise dans la cavité osseuse et de reconnaître l'instant où la profondeur de cette cavité correspond à la profondeur souhaitée, profondeur qui peut varier selon l'implant.

Le dessin annexé représente, à titre d'exemple, deux modes d'exécution de l'invention.
La figure 1 est une vue de l'intérieur d'une fraise hémisphérique selon le premier mode d'exécution.
La figure 2 est une vue de profil de la fraise représentée à la figure 1.
La figure 3 est une vue selon l'axe de rotation de cette même fraise, montrant ses moyens de fixation à un porte-fraise.
La figure 4 représente une variante d'exécution de la fraise représentée aux figures 1 à 3.
La figure 5 est une vue de l'intérieur d'une fraise tronconique selon le second mode d'exécution.
La figure 6 est une vue de profil de ce second mode d'exécution.

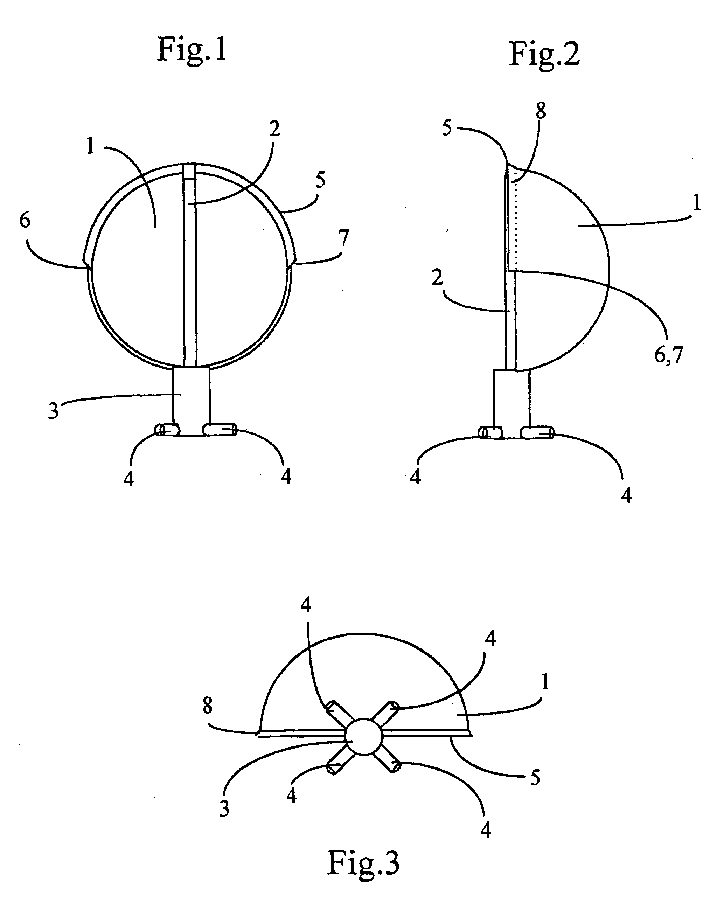

La fraise représentée aux figures 1 à 3 est constituée essentiellement d'une calotte hémisphérique creuse 1 en acier munie d'une barre diamétrale de rigidification 2 et rigidement solidaire d'un plot cylindrique 3 munis de quatre tenons 4 dirigés radialement de manière à former une croix pour la fixation de la fraise à un porte-fraise tel que décrit dans le brevet EP 0 704 191 du demandeur. L'axe du plot 3 détermine l'axe de rotation de la fraise qui coïncide, dans le mode d'exécution représenté, avec l'axe de la barrette 2.

La surface extérieure de la calotte 1 est lisse et peut être rectifiée. Le bord de la calotte 1 se présente comme une arête tranchante 5 sur la moitié de la circonférence du bord opposé aux moyens de fixation 3, 4 au porte-fraise. L'arête de coupe 5 est donc située dans un plan contenant l'axe de rotation de4 la fraise. Comme pour toute fraise, une dépouille 8 est nécessaire. Celle-ci est obtenue en évasant légèrement le bord de la calotte sur la partie affûtée.

Le bord de la calotte 1 présente en outre deux décrochements 6 et 7 diamétralement opposés et situés aux extrémités de l'arête tranchante 5. Le bord de la calotte 1 n'est donc pas entièrement dans un même plan.

En opération, la fraise peut être entraînée dans un sens ou dans l'autre. Lors de cette rotation, c'est l'une ou l'autre des moitiés de l'arête 5 qui travaille. Les décrochements 6 et 7 permettent de visualiser la pénétration de la fraise dans la cavité osseuse. Les décrochements pourraient donc être remplacés par tout autre repère permettant la même visualisation.

Comme ceci a déjà été mentionné, la surface de la calotte pourrait présenter une ou plusieurs dents comme les fraises de l'art antérieur.

Les moyens de fixation de la fraise au porte-fraise pourraient bien entendu être différents et adaptés au porte-fraise utilisé.

Le bord de la fraise pourrait présenter un seul décrochement situé dans le plan équatorial de la sphère reconstituée.

La calotte hémisphérique pourrait être légèrement inclinée relativement à l'axe de rotation dans le but de former une cavité légèrement non sphérique, ceci afin d'obtenir une certaine tension sur la prothèse. Dans ce cas, l'axe de rotation n'est plus situé dans le plan méridien limitant la calotte et l'arête de coupe 5 est située dans un plan légèrement incliné relativement à l'axe de rotation.

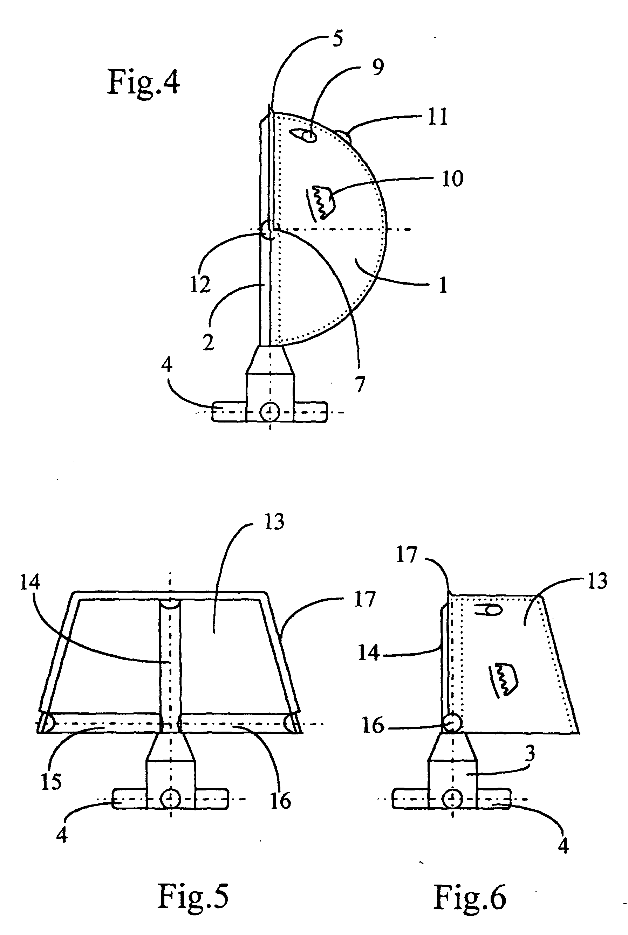

La surface de la calotte pourrait présenter des dents de coupe et d'appui. La figure 4 représente des exemples de dents. Ces dents pourraient avoir la forme de la dent 9, c'est-à-dire une forme conventionnelle obtenue par découpage et repoussage du métal vers l'extérieur. Ces dents 9 sont réparties sur la surface de la fraise et servent non seulement de dents de coupe, mais avant tout d'appui assurant un dégagement en arrière de l'arête de coupe 5, dégagement empêchant un bourrage.

Ces dents de coupe et d'appui pourraient également avoir la forme représentée en 10, forme particulière décrite dans la demande de brevet suisse N° 1 196/97.

Au lieu de prévoir des dents d'appui, il serait possible de prévoir des dômes d'appui, tels que le dôme 11, c'est-àdire une surface formant une sorte de bulle à la surface extérieure de la fraise. Dans la variante représentée à la figure 4, la fraise comporte en outre une deuxième barre d'armature 12, plus précisément deux demi-barres perpendiculaires à la barre principale 2.

Les figures 5 et 6 représentent un autre mode d'exécution de la fraise selon l'invention. Dans ce second mode d'exécution, le corps creux est en forme de demi-tronc de cône 13 supporté par une barre axiale 4 coïncidant avec l'axe de rotation de la fraise et deux barres transversales 15 et 16 perpendiculaires à la barre 14. Le bord du corps creux 13 forme une arête de coupe 17 semblable à l'arête 5 du premier mode d'exécution. Comme la fraise hémisphérique, la fraise tronconique peut présenter des dents d'appui ou des dômes d'appui répartis sur sa surface. Une telle fraise tronconique sera utilisée pour la préparation d'une cavité osseuse destinée à recevoir un implant.

## Revendications

1. Fraise chirurgicale, destinée en particulier au façonnage de la cavité cotyloïdienne lors d'un remplacement par prothèse totale de l'articulation de la hanche, en forme de corps creux de révolution muni de moyens de fixation (3, 4) à un porte-fraise pour son entraînement en rotation, le corps creux (1 ; 13) étant supporté par une barre (2 ; 14), **caractérisée en ce que** l'axe de la barre coïncide au moins approximativement avec l'axe de rotation de la fraise, que le corps creux s'étend essentiellement d'un côté d'un plan contenant l'axe de ladite barre et qu'une partie au moins de la longueur du bord du corps creux forme une arête de coupe (5 ; 17).

2. Fraise selon la revendication 1, **caractérisée en ce que** le corps creux est en forme de calotte (1) découpée dans une sphère.

3. Fraise selon la revendication 2, **caractérisée en ce que** la calotte (1) est essentiellement hémisphérique.

4. Fraise selon la revendication 3, **caractérisée en ce que** l'arête de coupe (5) s'étend dans un plan contenant l'axe de rotation de la fraise ou dans un plan parallèle audit axe.

5. Fraise selon la revendication 3, **caractérisée en ce que** l'arête de coupe (5) s'étend dans un plan légèrement incliné relativement à l'axe de rotation.

6. Fraise selon l'une des revendications 2 à 5, **caractérisée en ce que** le bord de la calotte (1) présente au moins un décrochement (6, 7) situé dans le plan équatorial de la sphère reconstituée par la rotation de la calotte.

7. Fraise selon la revendication 1, **caractérisée en ce que** le corps creux est en forme de demi-tronc de cône (13).

8. Fraise selon l'une des revendications 1 à 7, **caractérisée en ce que** la surface extérieure du corps creux est entièrement lisse.

9. Fraise selon l'une des revendications 1 à 7, **caractérisée en ce que** la surface extérieure du corps creux présente au moins une dent de coupe et d'appui (9 ; 10).

10. Fraise selon l'une des revendications 1 à 7, **caractérisée en ce que** la surface extérieure du corps creux présente au moins un dôme d'appui (11).

## Patentansprüche

1. Chirurgischer Fräser, der insbesondere zur Formgebung der Gelenkpfanne beim Ersetzen des Hüftgelenks durch eine Gesamtprothese bestimmt ist und welcher die Form eines hohlen Umdrehungskörpers hat, der mit Befestigungsmitteln (3, 4) zur Befestigung an einer Frässpindel versehen ist, mit der er in Drehung versetzt werden kann, wobei der Hohlkörper (1; 13) von einem Stab (2; 14) getragen wird, **dadurch gekennzeichnet, dass** die Achse des Stabs wenigstens näherungsweise mit der Drehachse des Fräsers zusammenfällt, dass sich der Hohlkörper im wesentlichen auf einer Seite einer die Achse des erwähnten Stabs enthaltenden Ebene erstreckt und dass wenigstens ein Teil der Länge des Rands des Hohlkörpers eine Schnittkante (5; 17) bildet.

2. Fräser nach Anspruch 1, **dadurch gekennzeichnet, dass** der Hohlkörper die Form einer aus einer Kugel geschnittenen Kalotte (1) hat.

3. Fräser nach Anspruch 2, **dadurch gekennzeichnet, dass** die Kalotte (1) im wesentlichen halbkugelförmig ist.

4. Fräser nach Anspruch 3, **dadurch gekennzeichnet, dass** sich die Schneidkante (5) in einer die Drehachse des Fräsers enthaltenden Ebene oder in einer parallel zur erwähnten Achse orientierten Ebene erstreckt.

5. Fräser nach Anspruch 3, **dadurch gekennzeichnet, dass** sich die Schneidkante (5) in einer zur Drehachse leicht geneigten Ebene erstreckt.

6. Fräser nach einem der Ansprüche 2 bis 5, **dadurch gekennzeichnet, dass** der Rand der Kalotte (1) wenigstens einen Absatz (6, 7) aufweist, der in der Äquatorialebene der durch Drehung der Kalotte wiederhergestellten Kugel liegt.

7. Fräser nach Anspruch 1, **dadurch gekennzeichnet, dass** der Hohlkörper die Form eines halben Kegelstumpfs (13) hat.

8. Fräser nach einem der Ansprüche 1 bis 7, **dadurch gekennzeichnet, dass** die Aussenfläche des Hohlkörpers vollständig glatt ist.

9. Fräser nach einem der Ansprüche 1 bis 7, **dadurch gekennzeichnet, dass** die Aussenfläche des Hohlkörpers wenigstens einen Schneid- und Auflagezahn (9; 10) aufweist.

10. Fräser nach einem der Ansprüche 1 bis 7, **dadurch gekennzeichnet, dass** die Aussenfläche des Hohlkörpers wenigstens eine Auflagekuppe (11) aufweist.

## Claims

1. A surgical reamer, intended in particular for the shaping of the cotyloid cavity in the event of a replacement of a hip joint by total prosthesis, in the form of a hollow body of rotation equipped with fixing means (3, 4) for attachment to a reamer spindle in order to be driven in rotation, the hollow body (1; 13) being supported by a bar (2; 14) **characterised in that** the axis of the bar coincides at least approximately with the axis of rotation of the reamer, the hollow body extends essentially on one side of a plane containing the axis of said bar, and at least part of the length of the rim of the hollow body forms a cutting edge (5; 17).

2. The reamer as claimed in claim 1, **characterized in that** the hollow body is in the form of a cap (1) cut out from a sphere.

3. The reamer as claimed in claim 2, **characterized in that** the cap (1) is essentially hemispherical.

4. The reamer as claimed in claim 3, **characterized in that** the cutting edge (5) extends in a plane containing the axis of rotation of the reamer or in a plane parallel to said axis.

5. The reamer as claimed in claim 3, **characterized in that** the cutting edge (5) extends in a plane slightly inclined relative to the axis of rotation.

6. The reamer as claimed in one of claims 2 to 5, **characterized in that** the rim of the cap (1) possesses at least one offset (6, 7) situated in the equatorial plane of the sphere as reconstituted by the rotation of the cap.

7. The reamer as claimed in claim 1, **characterized in that** the hollow body is in the shape of a half-frustum (13) .

8. The reamer as claimed in one of claims 1 to 7, **characterized in that** the outer surface of the hollow body is entirely smooth.

9. The reamer as claimed in one of claims 1 to 7, **characterized in that** the outer surface of the hollow body possesses at least one cutting and support tooth (9; 10).

10. The reamer as claimed in one of claims 1 to 7, **characterized in that** the outer surface of the hollow body possesses at least one support dome (11).
